# DEMANDE DE BREVET EUROPEEN

(11) **EP 1 162 453 A1**
(43) Date de publication de la demande: **12.12.2001**
(21) Numéro de dépôt: 00202008.9
(22) Date de dépôt: 07.06.2000
(51) Int. Cl.: G01N 27/30, C12Q 1/00

(54) **Capteur électrochimique à reproductibilité accrue**

(71) Demandeur: ASULAB S.A., CH-2501 Bienne (CH)
(72) Inventeur: Jaeger, Gérard, 1807 Blonay (CH)
(74) Mandataire: Thérond, Gérard Raymond

(57) **Abrégé**

L'invention concerne un capteur permettant de déterminer la concentration d'un composant et étant formé par une languette de petites dimensions comprenant un substrat (1) plastique mince supportant au moins deux bandes conductrices de courant (4, 5) séparées par une étroite bande (3) isolante, ledit substrat (1) et lesdites bandes (4, 5) étant recouverts d'un revêtement (2) plastique dans lequel sont découpées, à une extrémité une ouverture (8) laissant apparaître des portions de bandes (4, 5) pour la connexion à un appareil électronique et à l'autre extrémité deux fenêtres (9a, 9b) séparées par une bande (11) du revêtement (2), lesdites fenêtres (9a, 9b) délimitant sur les bandes (4, 5) les surfaces utiles d'une électrode de référence en dessous d'une première fenêtre (9b) et d'une électrode de mesure en dessous d'une deuxième fenêtre (9a) revêtue d'un réactif spécifique (10) du composant. Il est caractérisé par le fait qu'au moins la fenêtre de mesure (9a) à un contour curviligne sans angle vif.

Application à un capteur de glucose dans lequel le réactif spécifique contient au moins la glucose oxydase (GOD) et un médiateur.

## Description

La présente invention a pour objet un capteur électrochimique en forme de languette destiné à mesurer, à l'aide d'un réactif préalablement déposé sur son électrode de travail, la concentration d'un composant dans un échantillon d'une solution ou d'un liquide d'origine naturelle ou biologique d'une façon reproductible d'un capteur à un autre capteur.

L'invention concerne plus particulièrement de tels capteurs utilisés pour le suivi médical d'un patient nécessitant la mesure fréquente d'un composant dans un fluide biologique corporel de façon à adapter un traitement en fonction d'une valeur de référence, tel que le taux de glucose dans le sang pour une personne souffrant du diabète.

Au cours des dix dernières années, les capteurs électrochimiques généralement jetables, destinés à des mesures biologiques ont connu un développement considérable visant à en améliorer les qualités, telles que la sensibilité, la fiabilité, la rapidité de réponse ou la facilité d'emploi par un usager. De façon générale, ces capteurs sont constitués par un support isolant de petites dimensions, supportant au moins deux bandes conductrices électriquement séparées et connectables à une extrémité à un appareil de mesure électronique, lesdites bandes conductrices étant recouvertes d'un film dans lequel sont découpées deux fenêtres découvrant des portions de bandes formant respectivement l'électrode de référence et l'électrode de travail sur laquelle est immobilisée une quantité déterminée de réactif spécifique du composant dont on veut déterminer la concentration. Après avoir déposé sur la zone de mesure l'échantillon à analyser, par exemple une goutte de sang, la mesure est effectuée de façon indirecte par l'exploitation d'un signal électrique engendré par l'interaction entre ledit réactif spécifique et ledit composant.

Cette exploitation du signal électrique consiste généralement en des mesures conductométriques, voltamétriques, ampérométriques, coulométriques ou polarographiques permettant à un appareil électronique de mesure d'interpréter ledit signal et d'afficher directement sur un écran la concentration du composant selon un mode déterminé (mg/dl, mmol/l). Pour que la valeur affichée soit toujours la même pour une concentration déterminée, il faut que le signal électrique fourni par le capteur ne varie pas en fonction du capteur utilisé, c'est-à-dire que ces capteurs puissent être fabriqués à faible coût en série pour être jetables par un procédé garantissant néanmoins un haut degré de reproductibilité. Les paramètres sur lesquels on peut agir sont notamment la précision du dosage des différents composés entrant dans la composition du réactif spécifique, la précision de la quantité de réactif déposée sur l'électrode de travail, et la précision de la surface utile des électrodes, en particulier de la surface de l'électrode de travail effectivement recouverte par le réactif spécifique. Il est relativement facile d'obtenir une grande précision pour la composition du réactif et pour la quantité déposée sur l'électrode de travail. En suivant les enseignements du brevet EP 0 787 984, il est également possible d'avoir une grande précision au niveau de la surface utile des électrodes en ayant des bandes conductrices qui traversent les fenêtres sans laisser apparente aucune portion du substrat. Par contre, la demanderesse s'est aperçue qu'il était relativement difficile d'avoir un recouvrement parfait et reproductible de la surface utile de l'électrode de travail par le réactif spécifique lorsqu'il est appliqué par pipetage dans des fenêtres ayant les formes actuelles, à savoir des fenêtres rectangulaire ou des fenêtres en forme de demi-lune, c'est-à-dire dans les deux cas des fenêtres ayant des contours présentant des angles vifs.

En effet le matériau dans lequel sont découpées les fenêtres est en général un matériau hydrophobe, tel que le polyéthylènétéréphtalate (PET). Ce phénomène hydrophobe entre en compétition avec les propriétés capillaires du réactif spécifique, de sorte qu'en déposant par pipetage la quantité voulue de réactif spécifique au centre de la fenêtre, l'étalement ne s'effectue pas de façon uniforme sur toute la surface utile de l'électrode de travail. On observe en particulier une couverture très irrégulière au niveau des angles vifs.

Pour y remédier, il est possible d'effectuer le dépôt du réactif spécifique par pipetage en commençant par suivre au plus près le contour de la fenêtre et/ou en donnant une certaine inclinaison à la pipette. On conçoit aisément qu'un tel procédé n'est guère applicable à une production en série dans laquelle les capteurs sont fabriqués en lots sur des substrats en plaque ou en bande avant d'être découpés pour être conditionnés de façon unitaire.

Pour rendre hydrophile le contour de la fenêtre, on a également essayé d'effectuer un traitement préalable par un alcool. On obtient effectivement une meilleure mouillabilité de la fenêtre, à tel point que le réactif spécifique se répand aussi sur la feuille de couverture en PET. Il est en effet difficile, voire impossible d'effectuer ce traitement hydrophile uniquement au niveau des bords verticaux de la feuille de couverture.

L'invention a donc pour objet de pallier les inconvénients de cet art antérieur en procurant un capteur électrochimique dont la surface utile de l'électrode de travail est recouverte de façon uniforme et reproductible par un réactif spécifique d'un composant présent dans une solution ou un liquide d'origine naturelle ou biologique, grâce à une forme oblongue donnée au moins à la fenêtre délimitant la surface utile de l'électrode de travail comportant ledit réactif spécifique.

D'autres caractéristiques et avantages de l'invention apparaîtront mieux à la lecture de la description détaillée qui suit, concernant à titre illustratif et non limitatif un capteur électrochimique pour déterminer un taux de glucose dans le sang, en référence aux dessins annexés dans lesquels :
- la figure 1 représente en perspective un capteur selon l'invention,
- la figure 2 représente en vue de dessus agrandie la zone de mesure du capteur de la figure 1.

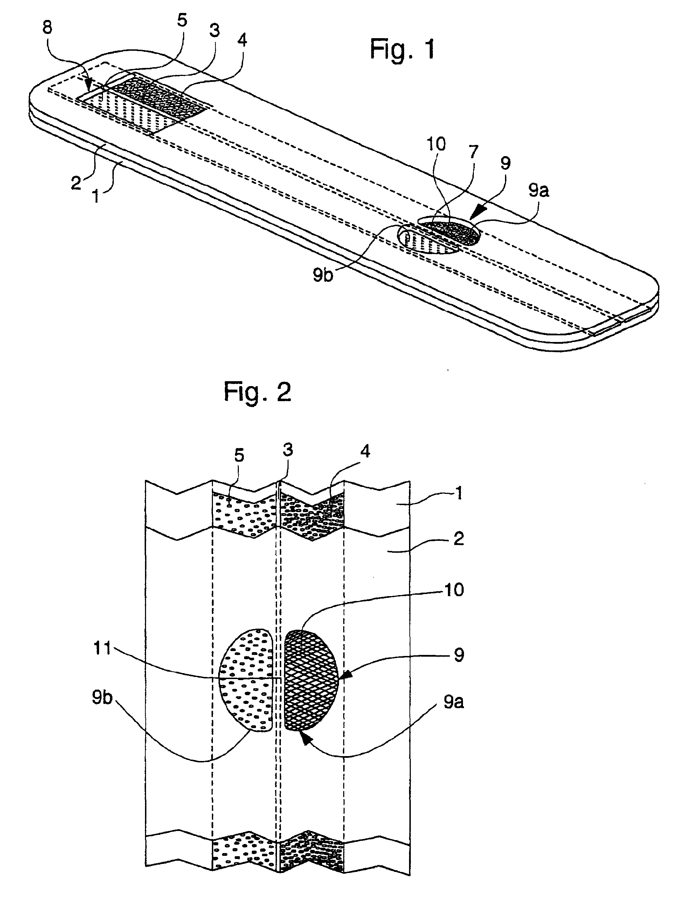

En se référant aux figures 1 et 2, on a représenté un capteur électrochimique pour mesurer le taux de glucose dans le sang, c'est à dire un capteur dont l'électrode de mesure est revêtue d'un réactif spécifique 10 dont la composition sera explicitée plus loin. Ce capteur a la forme d'une languette mince de petites dimensions d'une épaisseur totale comprise entre 0,40 et 0,80 mm, de préférence environ 0.60 mm, d'une largeur comprise entre 6 et 12 mm de préférence environ 8 mm et d'une longueur de l'ordre de 40 mm.

Le capteur comprend un substrat 1 mince isolant, par exemple obtenu à partir d'une feuille ou d'une bande de polyéthylènethéréphtalate (PET). Le substrat supporte deux bandes conductrices 4, 5 électriquement isolées par une étroite bande 3 du substrat 1. La nature des bandes conductrices 4, 5 et leur mode d'application sur le substrat 1 sont bien connus de l'homme de l'art. Le procédé préféré dans le cadre de la présente invention consiste à laminer à chaud deux films isolants ayant une surface métallisée pour la bande conductrice 4 qui formera l'électrode de travail 9a et pour la bande conductrice 5 qui formera l'électrode de référence 9b. Ces deux films métallisés peuvent être identiques ou avoir au contraire des revêtements métalliques différents. On utilise par exemple le couple bien connu Pt ou Pd - Ag/AgCl. L'ensemble est recouvert d'un revêtement isolant 2 dans lequel sont découpées deux zones 8, 9 laissant apparaître des portions de bandes conductrices 4, 5. Une première zone 8, située à une extrémité du capteur, permet de connecter lesdits bandes conductrices 4, 5 à l'appareil électronique de mesure. Une deuxième zone 9 constitue la zone de mesure sur laquelle sera déposée une goutte de sang à analyser. Elle comprend deux fenêtres situées respectivement au-dessus de portions de bandes 4, 5, une première fenêtre 9a délimitant l'électrode de travail et une deuxième fenêtre 9b délimitant l'électrode de référence, sans laisser apparente aucune position du substrat 1. Ces deux fenêtres 9a, 9b sont séparées par une bande 11 du revêtement isolant 2.

Les deux fenêtres 9a, 9b sont caractérisées par le fait qu'elles ont un contour curviligne ne présentant aucun angle vif. Dans le mode de réalisation préféré représenté à la figure 2 on voit que les fenêtres 9a, 9b ont une configuration en "grain de café", c'est à dire inscrite dans un cercle de sorte qu'une goutte de sang déposée dans cette zone les recouvre parfaitement et que la jonction ionique soit facilitée par le rapprochement des bords en regard. Cette configuration permet donc de déposer par pipetage une quantité déterminée de réactif spécifique 10 sensiblement au milieu de la fenêtre de mesure 9a et d'avoir un recouvrement parfait de toute la surface utile de l'électrode de travail et sans débordement au-delà de ladite fenêtre. Dans l'exemple choisi concernant la détermination d'un taux de glucose le réactif spécifique comprend notamment la glucose oxydase (GOD) et un médiateur permettant de transférer les électrons, par exemple un des médiateurs décrits dans le brevet US 5,378,628, à savoir des complexes de mono, bis ou tris 2-2'-bipyridines de ruthénium, d'osmium ou de vanadium, dans lesquels au moins un des ligands bipyridine est substitué par au moins un groupe donneur d'électrons.

Lorsque les fenêtres sont réalisées par étampage, ce qui constitue le mode de réalisation préféré, on a malgré tout souvent observé la formation de "cheveux d'ange" dans les angles vifs, c'est à dire de très fins filaments qui peuvent également être responsable de la mauvaise répartition du réactif spécifique 10 à cet endroit. Avec le contour curviligne selon l'invention, cet inconvénient est totalement éliminé.

La description précédente a été faite en référence à un capteur électrochimique pour la détermination d'un taux de glucose, mais l'homme de métier peut, sans sortir du cadre de l'invention, faire les adaptations nécessaires pour tout autre type de capteur électrochimique pour déterminer ou mesurer d'autres paramètres chimiques ou biologiques.

## Revendications

1. Capteur électrochimique pour déterminer la concentration d'un composant présent dans une solution ou dans un liquide d'origine naturelle ou biologique, formé par une languette de petites dimensions comprenant un substrat (1) plastique mince supportant au moins deux bandes conductrices de courant (4, 5) séparées par une étroite bande (3) isolante du substrat (1), ledit substrat (1) et lesdites bandes (4, 5) étant recouverts d'un revêtement (2) plastique dans lequel sont découpées, à une extrémité une ouverture (8) laissant apparaître des portions de bandes (4, 5) pour la connexion à un appareil électronique et près de l'autre extrémité deux fenêtres (9a, 9b) séparées par une bande (11) du revêtement (2), lesdites fenêtres (9a, 9b) délimitant sur les bandes (4, 5) les surfaces utiles d'une électrode de référence en dessous d'une première fenêtre (9b) et d'une électrode de mesure en dessous d'une deuxième fenêtre (9a) destinée à être revêtue d'un réactif spécifique (10) du composant dont on veut déterminer la concentration, **caractérisé en ce qu'**au moins la fenêtre de mesure (9a) à un contour curviligne sans angle vif.

2. Capteur électrochimique selon la revendication 1, **caractérisé en ce que** la fenêtre de référence (9b) a également un contour curviligne.

3. Capteur électrochimique selon la revendication 2, **caractérisé en ce que** la fenêtre de mesure (9a) et la fenêtre de référence (9b) sont symétriques par rapport à l'étroite bande isolante (3) séparant les bandes conductrices (4, 5).

4. Capteur électrochimique selon la revendication 3, **caractérisé en ce que** la fenêtre de mesure (9a) et la fenêtre de référence (9b) ont une configuration en grain de café.

5. Capteur électrochimique selon la revendication 1 pour la détermination d'un taux de glucose dans le sang, **caractérisé en ce que** le réactif spécifique contient au moins la glucose oxydase et un médiateur chimique susceptible de transférer les électrons.

6. Capteur électrochimique selon la revendication 5, **caractérisé en ce que** le médiateur est choisi parmi les complexes mono, bis ou tris 2-2'-bipyridine de ruthénium, d'osmium ou de vanadium dans lesquels au moins un des ligands bipyridine est substitué par au moins un groupe donneur d'électrons.

7. Capteur électrochimique selon la revendication 1, **caractérisé en ce que** le réactif spécifique (10) est déposé par pipetage dans la fenêtre de mesure (9a).
